# EUROPEAN PATENT SPECIFICATION

(11) **EP 2 173 474 B1**
(45) Date of publication and mention of the grant of the patent: **05.08.2015**
(21) Application number: 08762641.2
(22) Date of filing: 20.06.2008
(51) Int. Cl.: B01J 19/00, B01L 3/00, C12M 1/20, C40B 40/08, C40B 40/10, C40B 50/14

(54) **METHOD FOR MAKING A MICROARRAY**
VERFAHREN ZUR HERSTELLUNG EINES MIKROARRAYS
PROCÉDÉ DE PRÉPARATION D'UN MICRORÉSEAU

(30) Priority: 20.06.2007 GR 20070100394
(43) Date of publication of application: 14.04.2010
(73) Proprietor: National Center for Scientific Research Demokritos, 15310 Athens (GR); Tserepi, Angeliki, 153 10 Agia Paraskevi Attikis (GR); Gogolidis, Evangelos, 153 10 Agia Paraskevi Attikis (GR); Kakabakos, Sotirios E., 15310 Aghia Paraskevi, Athens (GR); Petrou, Panagiota, 153 10 Agia Paraskevi Attikis (GR)
(72) Inventor: TSEREPI, Angeliki, GR-15310 Agia Paraskevi Attikis (GR); GOGOLIDES, Evangelos, GR-15310 Agia Paraskevi Attikis (GR); KAKABAKOS, Sotirios, GR-15310 Agia Paraskevi Attikis (GR); PETROU, Panagiota, GR-15310 Agia Paraskevi Attikis (GR); BAYIATI, Pinelopi, GR-15310 Agia Paraskevi Attikis (GR); MATROZOS, Evrimahos, GR-15310 Agia Paraskevi Attikis (GR)
(74) Representative: Malamis, Alkisti-Irene
(86) International application number: PCT/GR2008/000048
(87) International publication number: WO 2008/155585

(56) References cited:
- EP-A- 1 364 702
- US-A1- 2003 082 587
- US-A1- 2005 130 226
- US-B1- 6 565 813

## Description

### Field of the invention

The invention relates to a method for making a carrier for a biomolecule microarray and to a method for making a biomolecule microarray. In particular, the invention relates to a method for making a carrier for a biomolecule microarray which enables the selective adsorption of proteins using selective plasma-induced deposition of a hydrophobic material.

### Background to the Invention

Microarrays have become an invaluable tool for large-scale and high-throughput bioanalytical applications. They allow fast, easy, and parallel detection of thousands of addressable elements in a single experiment. They have been used for basic research, diagnostics and drug discovery. Their significance and future applications have been reviewed extensively in the literature (M. Cretich et. al., Biomolecular Engineering 23, 77 (2006), S. Venkatasubbarao Trends in Biotechnology 22(12) (2004), D. S. Wilson et. al., Current Opinion in Chemical Biology 6, 81 (2001), H. Zhu et.al., Current Opinion in Chemical Biology 7, 55 (2003)). Microarrays consist of immobilized biomolecules spatially addressed/arranged on surfaces such as planar surfaces (usually modified microscope glass slides), microchannels or microwells, or arrays of beads. Biomolecules commonly immobilized on microarrays include oligonucleotides, polymerase chain reaction (PCR) products, proteins, lipids, peptides and carbohydrates. Ideally, the immobilized biomolecules must retain activity, remain stable, and not desorb during reaction and washing steps. The immobilization procedure must ensure that the biomolecules are immobilized at optimal density to the microarray surface to provide efficient binding of the counterpart molecules in the sample. Reduced autofluorescence of the solid substrate and minimal non-specific binding are also important criteria for high quality array fabrication.

High density protein microarrays (>30000 protein spots per slide) can be achieved using robotic contact printing tools, such as those developed for creating DNA microarrays (G. MacBeath, S. L. Schreiber, Science 289, 1760 (2000) and H. Zhu et. al., Science 293, 2101 (2001)). The contact printing arrayers deliver subnanoliter sample volume directly to the surface using tiny pins. A disadvantage of these contact printing robots is the need to touch the surface often leading to pin damage. Therefore, non-contact robotic printers, which use ink-jet technology, were developed to deposit nanoliter to picoliter protein droplets to polyacrylamide gel packets (P. Arenkov et. al., Anal. Biochem.. 278, 123 (2000)) and nanowells (on a polydimethylsiloxane (PDMS) surfaces supported by the standard glass slides) (H. Zhu et. al., Nat. Genet. 26, 283 (2000)). Alternatively, electrospray deposition technology has been applied to deliver dry proteins to a dextran-grafted surface (N. V. Avseenko et. al., Anal. Chem. 74, 927 (2002)). This technology further reduced the spot size from ∼150 µm to ∼30 µm. A group at Purdue University used electrospray ionization of a protein mixture followed by mass ion separation and sequential soft landing deposition onto a surface to create a protein array (Z. Ouyang et. al., Science 301, 1351 (2003)). A major disadvantage of all these spotting approaches is the irregular shape and inhomogeneous intensity distribution inside the spots as well as between spots. These irregularities hamper the quantification of the spots using image processing programs and obstacle the full-automation of the arrays readout (G.-J. Zhang et al., J. Fluorescence 14, 369 (2004)). An ideal method should provide an array of highly regular spots concerning their positions and geometries as well as the probe distribution across each spot.

In order to surpass the shape irregularities of the spots, other approaches for creation of arrays have been devised, which include photochemical techniques, µ-contact printing, microfluidic networks and photolithography. The photochemical method uses chemically labile species that are activated upon UV irradiation, to bind target molecules (H. Sigrist et. al., Optic. Eng. 34(8), 2339 (1995)). Disadvantages of this method are protein deactivation and the fact that continuous irradiation of the photoactivated material limits the number of different molecules that can be immobilized on different sites on the same surface. Micro-contact printing uses elastomeric stamps to print molecules on self-assembled monolayers (SAMs) of alkanethiols on gold (R. S. Kane et. al., Biomaterials 20, 2363 (1999)). This method suffers in terms of the uniformity and repeatability of the resulted spots, the number of depositions that could be achieved with the same stamp and the difficulty to deposit multiple proteins on the same substrate. In the case of microfluidic networks proteins are applied to a surface using microfluidic channels, resulting in stripes of immobilized capture agents. Orthogonal parallel channels are then created to deliver samples that intersect with the original stripes, producing fluorescent signal when the immobilized proteins bind to the sample (C. A. Rowe et. al., Anal. Chem. 71, 433 (1999), A. Bernard et. al., Anal. Chem. 73, 8 (2001) and E. Delamarche et. al., Science 276, 779 (1997)). This method cannot be used for the creation of very small dimension patterns.

Photolithography can be used to generate patterns by photoablating proteins preadsorbed to a silicon or glass surface (J. A. Hammarbac et. al., J. Neurosci. Res. 13, 213 (1985)), by immobilizing proteins on thiol-terminated siloxane films that have been patterned by irradiation with UV light (S. K. Bhatia et. al., J. Am. Chem. Soc. 114, 4432 (1992)), and by covalently linking proteins to photosensitive groups (T. Matsouda et. al., US Patent 1993, 5,202,227). On the other hand, although photolithography involving conventional photoresist films, which is extensively used in microelectronics industry, could provide high definition patterns, it involves procedures not compatible with biomolecules such as exposure to UV radiation, organic solvents and processes at high temperatures. A photolithographic method has been developed in the Institute of Microelectronics in collaboration with the Institute of Radioisotopes & Radiodiagnostic Products of NCSR "Demokritos" which uses a biomolecule friendly process for the patterning of microarrays of different proteins on the same substrate through successive depositions (A. Douvas et. al., Biosens. Bioelectron. 17, 269 (2002)). This method results in spot dimensions of 5-10 µm but it has restrictions concerning the number of the successive applications of different biomolecules due to photoresist limitations.

One of the first technical challenges with protein microarrays is to fix a protein to arrays in a biologically active form. The simplest way to bind a protein onto a surface is through surface adsorption. This approach is based on adsorption of the macromolecules either by electrostatic forces on charged surfaces (for example on poly-lysine coated slides, B. B. Haab et. al., Genome Biol. 2(2), 4 (2001)) or by hydrophobic interactions (for example on nitrocellulose, T. O. Joos et. al., Electrophoresis 21, 2641 (2000) and H. Ge, Nucleic Acids Res. 28, e3 (2000)) or on polyvinylidene fluoride (PVDF) membranes ( K. Bussow et. al., Nucleic Acids Res. 26(21), 5007 (1998), D. J. Cahill, J. Immunol. Methods 250, 81 (2001) and G. Walter et. al., Curr. Opin. Microbiol. 3, 298 (2000)).

Another approach to immobilize proteins is through covalent binding. Proteins are often immobilized on modified glass surfaces (B. Guilleaume et. al., Proteomics 5(18), 4705 (2005)). This method requires the presence of reactive groups on the support (usually electrophilic groups such as epoxides (H. Zhu et. al., Nat. Genet. 26(3), 283 (2002)), aldehydes (G. MacBeath, P. L. Schreiber, Science 289, 1760 (2000)), succinimidyl esters/isothiocyanate functionalities (R. Benters et. al, Chembiochem 2(9), 686 (2001)) able to react with nucleophilic groups (amino, thiol, hydroxyl) on the ligand molecules. The functional groups on the surface are introduced by glass modification with organosilanes such as 3-glycidoxypropyltrimethoxysilane (GOPS) or 3-aminopropyltriethoxysilane (APTES). Alternatively, they can be inserted on more complex molecular architectures such as self-assembled monolayers (SAMS) (M. Schaeferling et. al., Electrophoresis 23(18), 3097, 2002)) or polymer grafted to the surface. Organosilanes can directly provide the functional groups for ligand attachment or react with a bifunctional ligand bearing the desired reactive group. A microarray surface has been developed (Y. Lee et. al., Proteomics 3(12), 2289 (2003)) with ProLinker™, a calixcrown derivative with a bifunctional coupling property that permits efficient immobilization of capture proteins on solid matrices such as gold films (B. T. Houseman et. al., Nat. Biotechnol. 20, 270 (2002) and C. Bieri et. al., Nat. Biotechnol. 17, 8105 (1999)) or aminated glass slides. Other substrates used for immobilization of biomolecules are various polymeric substrates such as poly(methyl methacrylate) (PMMA), polystyrene, cyclic olefin polymers or polycarbonate (F. Fixe et. al., Nucleic Acids Research 32(1), e9 (2004), A. Hozumi et. al., J. Vac. Sci. Technol. A 22(4), 1836 (2004), J. Kai et. al., 7th International Conference on Miniaturized Chemical and Biochemical Analysis Systems, 2003 & related patent US2005130226, Y. Feng et. al., Clinical Chemistry 50(2), 416 (2004)).

The use of a matrix that embeds the protein in a structured environment is an alternative way to immobilize proteins. This mechanism is based on the physical entrapment of proteins in gels such as polyacrylamide (P. Arenkov et. al., Anal. Biochem. 278(2), 123 (2000) and D. Guschin et. al., Anal. Biochem. 250, 203 (1997)) or agarose (V. Afanassiev et. al., Nucleic Acids Res. 28(12), e66 (2000)).

In spite of their simplicity, most adsorption methods present several drawbacks one of which is the high background level due to protein adsorption on non-designated areas. This is overcome, in some studies, by spatial modification of the substrate, and adsorption of proteins only onto the hydrophilic areas (European Patent 1364702A2, J. Damon Hoff et. al., Nano Letters 4(5), 853 (2004), S.-H. Lee et. al., Sensors and Actuators B 99, 623 (2004), A. Hozumi et. al., J. Vac. Sci. Technol. A 22(4), 1836 (2004), V. C. Rucker et. al., Langmuir 21, 7621 (2005)) or hydrophobic areas (J. Kai et al., 7th International Conference on Miniaturized Chemical and Biochemical Analysis Systems, 2003 and related patent US2005130226). Usually the selective modification is achieved by plasma treatment but in most cases the treated surfaces need further chemical modification for inducing protein adsorption. For example in EP1364702A2, the processing the surface of the hydrophilic binding sites is described as required (for example, with APTES) in order to increase the affinity of biomolecules to the hydrophilic sites.

It is an object of the invention to overcome or mitigate at least some of the problems outlined above.

### Summary of the invention

It is an object of this invention to develop a simple new method of fabricating hydrophilic/hydrophobic patterns on surfaces, with distinct biomolecule adsorption capabilities, by means of selective plasma etching/deposition technique. Under appropriate plasma conditions, patterned surfaces interact with the plasma environment, and areas of distinct wettability are produced. Biomolecules are adsorbed onto the hydrophilic areas, leaving the hydrophobic areas clean. The invention provides a method for fabrication of biomolecule, for example protein, microarrays containing thousands of spots fabricated in a single step, with a simple immersion of hydrophilic/hydrophobic patterned substrate in a bio-solution. The invention also provides a method for fabrication of multiple-biomolecule microarrays using commercial robotic spotters. It is another object of the invention to provide a method for making microarrays with minimum spot size of the order of 1 µm (or smaller) and thus of very high spot density. It is yet another object of the invention to use glass for the fabrication of hydrophilic/hydrophobic patterns to be used for the realization of biomolecule microarrays.

The process described above can be applied for a) hydrophobic/hydrophilic substrate patterning by selective fluorocarbon plasma deposition, b) selective biomolecule adsorption on hydrophilic versus hydrophobic areas by simple immersion of such patterned substrate in bio-solution and formation of thousands of spots of immobilized biomolecules in one step, c) fabrication of hydrophilic/hydrophobic patterned substrates in combination with robotic spotters for multiple-biomolecule microarrays creation, d) fabrication of microrrays with spot size of the order of tens of nm (depending on the resolution of the lithographic method used), leading to increased spot density, reduced reagent volumes and improvement of the statistical analysis of the immobilized biomolecules detection, and e) fabrication of microarrays without any non-specific binding, leading to increased signal to noise ratio.

According to the present invention there is provided a method for making a biomolecule microarray as defined in the appended independent claim 1. Further preferable features are defined in the appended dependent claims.

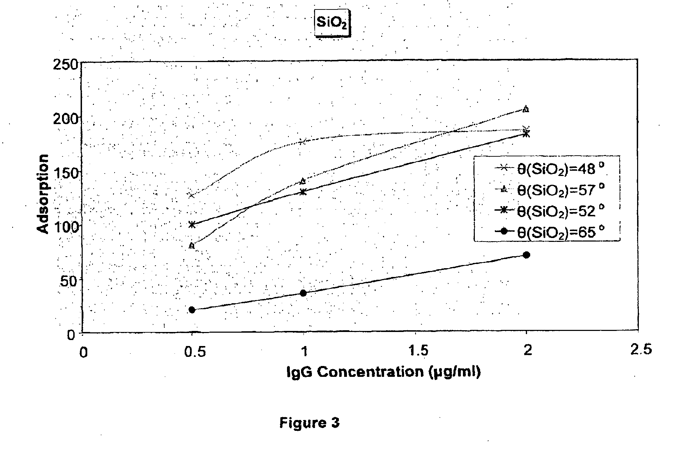

In respect to the method described in EP1364702A2, the method of the present differs advantageously in two points:
**1)** In the present invention the hydrophobic layer is not deposited on all the surface of the substrate, while this is the case in the method of EP1364702A2. On the contrary, according to the present invention the hydrophobic layer is deposited selectively only on the Si areas of the substrate. Therefore a lithographic and etching process are not required for patterning. As a result of that, neither the activation step (step 3 in Fig. 3) is required to allow deposition of the photoresist on the hydrophobic layer, nor the heating step (step 7 in Fig. 3) is required for the film to recover its hydrophobic properties. Therefore, the method proposed with the present invention is faster and simpler and it includes two steps less, compared to EP1364702A2.
**2)** In the present invention, since the hydrophilic areas are exposed to the plasma, proper plasma-induced chemical modification of these areas induces biomolecule adsorption, without the need for further chemical modification of the hydrophilic areas. In the contrary, further chemical modification of the hydrophilic areas is needed in EP1364702A2, to increase the affinity of biomolecules to the hydrophilic binding sites.

### Detailed description

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In a first aspect, the invention relates to a method for making a carrier for use in a biomolecule microarray having a patterned surface with a plurality of hydrophobic and hydrophilic areas, the method comprising
a) providing a substrate;
b) generating a patterned surface on the said substrate;
c) contacting the patterned surface with a plasma wherein a hydrophobic material is selectively deposited on selected areas of the surface of the substrate and the rest of the surface of the substrate is etched.
d) contacting the said patterned surface exposed to plasma with a biomolecule solution, or with a plurality of biomolecule solutions, wherein the biomolecule is a protein.

The term "carrier" as used herein refers to a solid support onto which the one or more biomolecule is immobilised. The support may be a membrane, glass slide or a chip, i.e. silicon chip.

The term "array" refers to an arrangement of entities in a pattern on a carrier or substrate. Although the pattern is typically a two-dimensional pattern, the pattern may also be a three-dimensional pattern. In a protein array, the entities are proteins and the term "protein microarray" as used herein refers to a protein microarray or a protein nanoarray.

As used herein, the term "substrate" refers to the bulk, underlying, and core material of the arrays. The substrate can be planar, e.g., have a horizontal plane in which the addresses are located at different discrete locations. The surface of the substrate can be flat (e.g., a glass slide) or can include indentations (e. g., wells) or partitions (e. g. barriers) or channels as in a microfluidic device. Examples of the substrates according to the invention are glass, Si, SiO₂ and Si₃N₄.

The term "biomolecule" as used herein refers to a protein, peptide, lipid, carbohydrate or nucleic acid. The nucleic acid may be cDNA, DNA or RNA. Preferably, the biomolecule according to the methods of the invention is a protein or peptide. As used herein, the word "protein" refers to a full-length protein or a portion of a protein. The protein may be a fusion protein and may comprise an affinity tag to aid in purification and/or immobilization. Proteins can be produced via fragmentation of larger proteins, or chemically synthesized. Proteins may be prepared by recombinant overexpression in a species such as, but not limited to, bacteria, yeast, insect, plant or animal cells. As used herein, the term "peptide" refers to a sequence of contiguous amino acids linked by peptide bonds. The peptides may be less than about twenty-five amino acids in length or alternatively, the peptide may be a "polypeptide" with at least about twenty-five amino acids linked by peptide bonds.

The term "area" according to the invention is used to refer to a microarray spots. Each microarray spot has a unique position and each spot corresponds to one or more specific biomolecule probe(s).

In one embodiment, the patterned surface is generated using a lithographic process, for example photolithography. Techniques used are further described in the examples.

In a preferred embodiment, deposition of the hydrophobic material and etching occur at the same time. In another preferred embodiment, the second substrate is hydrophilised.

In one embodiment, the first substrate is Si and the second substrate SiO₂ or Si₃N₄. SiO₂ may be deposited as a thin film on Si. A patterned surface of SiO₂ lines can then be formed using photolithographic and etching techniques. In another embodiment the substrate material is glass and the patterned thin film is a photoresist.

In one embodiment, the plasma may be supplied with a fluorocarbon gas. The term plasma refers to an ionized gas; that is, any gas containing ions and electrons. The plasma is a fluorocarbon plasma. Preferred fluorocarbon plasmas are those with a high concentration of radical CF₄ species, such as C₄F₈, CHF₃ or a mixture of CHF₃/CH₄.

Without wishing to be bound by theory, the inventors believe that during plasma treatment, the substrate is chemically modified to incorporate groups (such as C=O), onto the SiO₂ or glass surface, which can then react with biomolecules. Thus, after the plasma treatment, the substrates possess areas of distinct wettability (hydrophilic/hydrophobic patterning). The wettability of a liquid is defined as the contact angle between a droplet of the liquid in thermal equilibrium on a horizontal surface. These substrates are subsequently immersed in a protein solution or protein solution droplets are deposited ( for example by means of a robotic spotter on the surface) without any further chemical (or other) modification. As shown in the examples, fluorescent images taken after washing and blocking of the substrates show that protein is adsorbed only on the hydrophilic SiO₂ or Si₃N₄ surfaces and not on the Si surfaces covered with the plasma-deposited hydrophobic fluorocarbon film.

In one embodiment, the substrates is patterned SiO₂ or Si₃N₄ on a Si surface, fabricated with standard lithographic and subsequent etching techniques, are treated in C₄F₈ plasma in a high density inductively coupled plasma (ICP) etcher, under conditions that result in etching of SiO₂ or Si₃N₄, whereas a thin hydrophobic fluorocarbon film is deposited on the Si surface.

Conditions used according to the invention that allow deposition of a hydrophobic material on one substrate surface and etching of the second substrate surface are given in the examples. For example, the fluorocarbon gas flow rate may be about 25 sccm, the gas pressure from 2 to 10 m Torr, power from 800 to 1500 Watt, bias voltage substrate from -100 to -250 Volts, substrate temperature 0 to -50 °C, preferably 0 °C , and process time 10 to 90 sec. The etching rate may be within the range of 70 to 270nm/min.

The patterned surface, resulting from a lithographic process and subsequent plasma treatment, does not require any further chemical or other modification for the biomolecule immobilization. The term "modfied" includes chemical modification, such as treatment with biotin, streptavidin to enable protein binding to the hydrophilic region. Thus, in a preferred embodiment, the patterned surface of the carrier is not further modified after plasma treatment and etching to allow immobilisation of the biomolecule. Thus, in one embodiment, the patterned substrate is capable of binding one or more biomolecule without being further modified. Preferably, the biomolecule is a protein and binds to the hydrophilic areas on the patterned surface. Thus, in one embodiment, the invention relates to a method for making a carrier for a protein microarray having a patterned surface with a plurality of hydrophobic and hydrophilic areas, the method comprising
a) providing a substrate;
b) generating a patterned surface on the said substrate;
c) contacting the patterned surface with a fluorocarbon a plasma wherein a hydrophobic material is selectively deposited on selected areas of the surface of the substrate and the rest of the surface of the substrate is etched.
wherein the hydrophilic areas of the patterned surface are capable of binding a plurality of proteins without being further modified to enable protein immobilisation.

In an alternative embodiment, the patterned surface is simply immersed in a bio-solution containing biomolecules which further modify the patterned surface, preferably the hydrophilic areas. The biomolecules may be biotin, streptavidin but other reactive groups, or cross-linkers known in the art for biomolecule, in particular protein, immobilisation are also envisaged.

According to the invention, biomolecule spots with a diameter of the order of tens of nm can be achieved, i.e. the invention enables the fabrication of carriers for microarrays and microarrays of much higher spot density than that created by today's industrial instruments for biomolecule spotting.

Selective immobilization eliminates undesirable binding in the surrounding the spot area, a crucial issue in microarray technology. Using the methods of the invention, thousands of spots can be formed on one substrate during this one step of immersion in a bio-solution. The presence of areas with distinct wettability and protein adsorption capability on the same substrate can be exploited for the fabrication of biomolecule microarrays with decreased spot size due to the fact that bio-molecule immobilization is restricted inside the plasma-modified hydrophilic areas. The spot size is determined by the initial substrate patterning and therefore it depends on the resolution of the lithographic step. Given that current state-of-the-art lithographic processes (or standard industrial lithographic processes used in microelectronics fabrication) provide structures in the order of 100 nm, spots with diameter less than 1 µm could be easily achieved. Reduced spot size will lead to microarrays with increased spot density. In addition, when using a robotic spotter to deposit the biomolecules, multiple spot deposition of the same protein will occur using just one nano-droplet. The latter is the result of the reduced spot size which can be many orders of magnitude smaller than the nano-droplet diameter. For example, a typical nano-droplet delivered by an industrial robotic spotter has a diameter of the order of 100 µm. The minimum spot size achievable by the methods of the invention can be of the order of 1 µm or even 100 nm. Therefore, by using just one typical nano-droplet, an array of 50x50 1µm-size protein spots or an array of 500x500 100 nm-size protein spots can be fabricated according to the methods of the invention. This will lead to further reduction of the required reagent volumes and to improvement of the statistical analysis of immobilized biomolecules detection. Furthermore, when using multiple biomolecule solutions on the robotic spotter, multiple-biomolecule microarrays can be realized with spot density and signal to noise ratio much higher than found in today's applications using other methods.

The spot size according to the invention in diameter may be within the range of about 100nm to about 1 mm, preferably, 100nm to 100µm, preferably 1 nm to 10 µm or 100nm to 1µm.

In another aspect, the invention relates to a method for making protein microarrays based on a dry etching/deposition process, comprising the following steps: (a) selection of a first substrate and a thin film second substrate wherein the second substrate may be a thin film and wherein the first and second substrate differ in that under appropriate conditions, etching and hydrophilization of one material is possible simultaneously with deposition of a hydrophobic layer on the other material, (b) use of a patterning process for the creation of a regularly patterned substrate consisting of a patterned thin film of the second substrate on the first substrate, (c) exposure of said patterned substrate to a depositing plasma under conditions appropriate for selective deposition of a hydrophobic material on one of the two materials simultaneously with etching and hydrophilization of the other material, so as to result in a hydrophobic/hydrophilic patterned substrate, and (d) exposure of said hydrophobic/hydrophilic patterned substrate to a protein solution so as to result in protein adsorption on the hydrophilic regions.

The first substrate may be Si and the second substrate may be SiO₂ or Si₃N₄. Furthermore, the first substrate may be glass and the second substrate may be a photoresist material. The fluorocarbon gas is as described herein. The conditions used are described herein.

In another aspect, the invention relates to a carrier for a biomolecule microarray obtainable by a method as described herein.

In a further aspect, the invention relates to a method for making a biomolecule microarray comprising making a biomolecule microarray carrier as described herein, exposing the carrier to a plurality of biomolecules and adsorbing the biomolecules to the patterned surface of the carrier. In one embodiment, the biomolecule is a protein and adsorbed to the hydrophilic areas.

In one embodiment, the exposure to the biomolecules comprises exposure to a protein solution. The patterned substrate is immersed in the protein solution, and adsorption of proteins occurs on the hydrophilic regions. One spot may adsorb one protein and thus protein arrays containing as many spots as the hydrophilic spots are created in one step.

In a further aspect, the invention relates to a biomolecule microarray obtainable by a method as described herein.

In a final aspect, the invention relates to a biomolecule microarray having a patterned surface with a plurality of hydrophobic and hydrophilic areas wherein the hydrophobic areas comprise a fluorocarbon film and the hydrophilic areas comprise hydrophilised SiO₂ or S₃N₄ wherein the hydrophilised SiO₂ or S₃N₄ is capable of binding proteins without being further chemically modified.

The invention will be further described with reference to the following nonlimiting figures and examples.

### Brief description of drawings

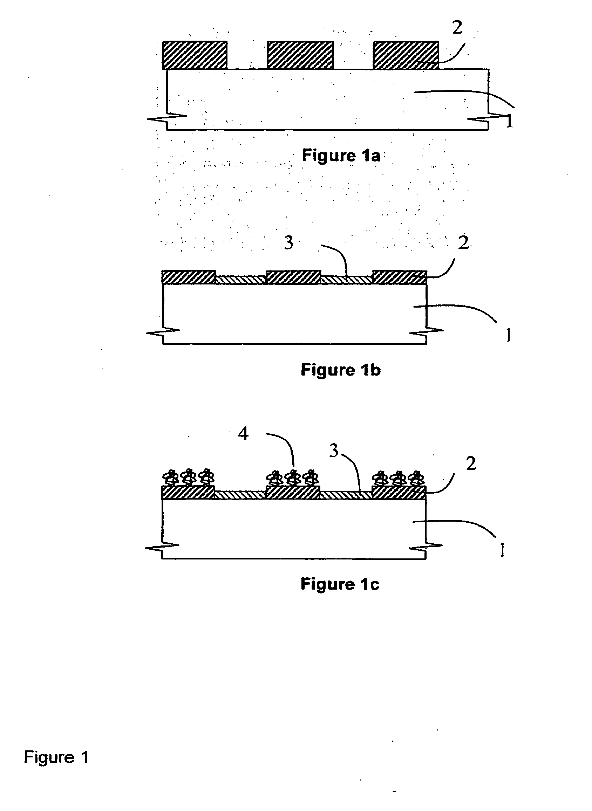

**Figure 1a** is a cross-sectional view of SiO₂/Si₃N₄ patterns (2) on Si surface (1) resulting from standard lithography and etching procedures.
**Figure 1 b** is a cross-sectional view of the patterned structure after C₄F₈ plasma treatment, where a thin hydrophobic fluorocarbon film (3) is selectively deposited on surface 1, and surface 2 is etched.
**Figure 1c** is a cross-sectional view of a C₄F₈ plasma-treated substrate, consisted of hydrophilic surfaces 2 and hydrophobic surfaces 3, after immersion of the substrate in a bio-solution. Biomolecules (4) are adsorbed onto hydrophilic surface 2 but not on hydrophobic surface 3.

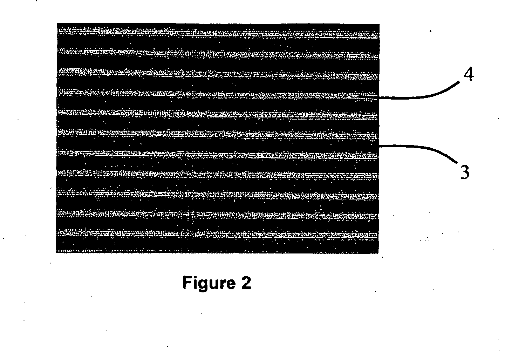

**Figure 2** is a fluorescent image of immobilized rabbit IgG lines visualized through reaction with anti-rabbit IgG labeled with AlexaFluor 488 (Molecular Probes Inc., Eugene, OR, USA). The rabbit IgG lines were created on a C₄F₈ plasma-treated substrate, consisted of hydrophilic SiO₂ lines 3 µm-wide on hydrophobic Si surface (3), after immersion of the substrate in rabbit IgG solution of 2 µg/ml and pH=7, incubation for 1 h, washing, and blocking of the surface with a 10 gr/L bovine serum albumin solution for 1 h, and washing of the surface. The visualization of the immobilized IgG was performed by immersion of the substrate in a 5 µg/ml AlexaFluor 488 labeled anti-rabbit IgG antibody solution of pH=7 for 1 h. Protein (4) is adsorbed onto hydrophilic surface 2 but not on hydrophobic surface 3.
**Figure 3** is a graph of the protein adsorption on hydrophilic SiO₂ surfaces 2 as a function of protein concentration for samples treated under different plasma conditions resulting in different SiO₂ hydrophilicities (expressed in terms of the water contact angle on such SiO₂ surfaces). The more hydrophilic a surface is (i.e. the lower contact angle), the higher is the protein adsorption.

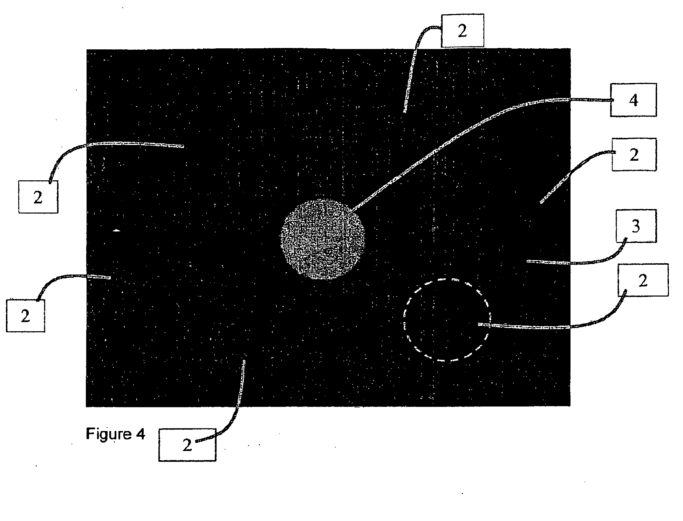

**Figure 4** is a fluorescent image of a C₄F₈ plasma-treated substrate, consisted of hydrophilic SiO₂ spots that are 1 mm wide (2) on hydrophobic Si surface (3), after deposition of a rabbit IgG solution droplet on just one SiO₂ spot, incubation, blocking (using bovine serum albumin), and deposition of a anti-rabbit IgG solution droplet. Protein (4) is adsorbed onto the hydrophilic spot, but not on the hydrophobic surface 3.

### Examples

### Example 1:

A Si substrate is used on which a thin film of SiO₂ is deposited. With conventional photolithography, using AZ 5214 as the photoresist mask, and subsequent wet etching of the exposed SiO₂ surface in BHF (NH₄F/HF/H₂O) solution, we form an array of SiO₂ lines, 3 µm wide (the same as the pattern dimension on the lithographic mask), on the Si substrate. (The process is schematically shown in Fig. 1a.) This sample is then treated in an inductively coupled plasma (ICP) reactor, under conditions that result in selective deposition of hydrophobic fluorocarbon film on Si, with simultaneous etching (not until etch end-point) of the hydrophilic SiO₂ patterns (Fig. 1b). These conditions in our experiments were: C₄F₈ gas, flow rate 25 sccm, gas pressure from 2 to 10 mTorr, power from 800 to 1500 Watt, bias voltage from -100 to -250 Volts, substrate temperature 0 °C, and process time 60-90 sec. The SiO₂ etching rates are in the range of 70-270 nm/min. However, we have previously achieved selective deposition also in CHF₃/CH₄ mixtures (P. Bayiati et. al. J.Vac. Sci. Technol. 2004). Water contact angles measured on the etched SiO₂ surfaces are 48-65 ° (hydrophilic surfaces) and on the Si surfaces covered with fluorocarbon film are 90-97 ° (hydrophobic surfaces).

After plasma treatment, the hydrophilic/hydrophobic patterned substrate is immersed in rabbit IgG solutions (Fig. 1c) of three different concentrations (0.5, 1, and 2 mg/ml) in 10 mM phosphate buffer (pH 7). After incubation for 1 hour at room temperature (RT), blocking with 10 mg/ml bovine serum albumin solution (BSA) (in 10 mM phosphate buffer, pH 7) and immersion in a solution containing 5 µg/ml AlexaFluor 488 labeled anti-rabbit IgG antibody (in 50 mM phosphate buffer, pH 7, containing 10 mg/ml BSA) for 1 hour at RT, the sample is observed with a fluorescent microscope. Figure 2 is a fluorescent image showing that protein is selectively adsorbed onto the hydrophilic SiO₂ patterns, without interacting with the Si surface covered with the thin hydrophobic fluorocarbon film. The protein adsorption is observed as a function of the protein concentration, and higher adsorption is observed for more hydrophilic surfaces (Fig. 3).

### Example 2:

In another case, Si₃N₄ instead of SiO₂ is used for the realization of hydrophilic patterns on hydrophobic Si. In this case, the conditions for selective etching/deposition are: C₄F₈ gas, flow rate 25 sccm, gas pressure from 2 to 5 mTorr, power from 800 to 1800 Watt, bias voltage from -150 to -250 Volts, substrate temperature 0 °C, and treatment time 11-15 sec. Under such conditions the etching rates of Si₃N₄ are in the range of 130-225 nm/min. Water contact angles measured on the Si₃N₄ surfaces are 77-81 ° (hydrophilic surfaces), and on the Si surfaces are 91-94 ° (hydrophobic surfaces). After immersion of the sample in protein solutions, following the procedure as in Example 1, fluorescent images show that protein is again selectively adsorbed only onto the hydrophilic plasma-treated Si₃N₄ surfaces.

### Example 3:

In another example, the method described above can be used for the formation of hydrophilic SiO₂ or Si₃N₄ spots on hydrophobic Si surfaces, with spot diameter of the order of 1 µm (or smaller depending on the resolution of the patterning method). A droplet of protein solution is then applied by means of a pipette only on one of the hydrophilic spots, following the procedure described in Examples 1 and 2, for protein immobilization and detection. The immobilization of the protein is indicated by the fluorescence image in Figure 4. Such substrates can be used for the fabrication of multiple-protein micro-arrays using a commercial robotic spotting system.

### Example 4:

In another example, a glass slide can be used as a substrate for the creation of the protein micro-arrays. In fact, we have shown previously (P. Bayiati, et. al. J. Vac. Sci. Technol. 2004) that under appropriate plasma conditions selective deposition of a fluorocarbon layer occurs on a photoresist surface simultaneously with etching of SiO₂ surface. Therefore, a glass substrate bearing photoresist patterns is an appropriate substrate where, by means of the method presented here, hydrophobic/hydrophilic patterning can be created and thus protein adsorption and spotting on the hydrophilic regions.

## Claims

1. A method for making a biomolecule microarray on a substrate having a patterned surface with a plurality of hydrophobic and hydrophilic areas, the method comprising
a) providing a substrate with a pattern on its surface;
b) contacting the patterned surface with a fluorocarbon plasma wherein a hydrophobic material is selectively deposited on selected areas of the surface of the substrate while, at the same time, the rest of the surface of the substrate is etched;
c) without any further modifications other than plasma exposure described in step b) above, contacting the said patterned surface exposed to plasma with a biomolecule solution, or with a plurality of biomolecule solutions, to enable adsorption of the biomolecule, wherein the biomolecule is a protein.

2. The method of claim 1 wherein the substrate is Si and the rest of the surface of the substrate is silicon dioxide SiO₂ or silicon nitride Si₃N₄.

3. The method of claim 1 wherein the substrate material is glass and the patterned thin film is a photoresist.

4. The method of any preceding claim wherein the fluorocarbon plasma is C₄F₈ or a mixture of CHF₃/CH₄.

5. The method of any preceding claim wherein the spot size is about 100nm to about 1mm in diameter.

6. The method of claim 5 wherein the spot size is about 100nm to about 100 µm in diameter.

7. The method of claim 5 wherein the spot size is about 100nm to about 10 µm in diameter.

8. The method of claim 5 wherein the spot size is about 100nm to about 1µm in diameter.

9. The method of any preceding claim wherein step b) is carried out using an inductively coupled plasma reactor.

10. The method of any preceding claim wherein in step b), the flow rate of the fluorocarbon gas is about 25 sccm, the gas pressure is about 2 to about 10m Torr, the power is about 800 to about 1500 Watt, the bias voltage is about -100 to about -250 V, the substrate temperature is about -50 to about 0 °C and the process time is about 10 to about 90 sec.

11. The method of any preceding claim wherein in step b), the etching rate is about 70 to 270nm/min for SiO₂ and about 130 to 250nm/min for Si₃N₄.

12. The method of any preceding claim wherein the biomolecule is adsorbed to the hydrophilic area.

13. The method of any preceding claim wherein exposure to a protein or peptide solution is carried out by means of a robotic spotter, in which case multiple proteins can be adsorbed or spotted on different hydrophilic regions.

## Patentansprüche

1. Methode um eine biomedizinische Microarray auf einem Objekt herzustellen, welches eine gemusterte Oberfläche mit einer Vielfalt an hydrophobischen und hydrophilen Bereichen haben soll, umfassend:
a) bereitstellung von einem Objekt mit einer gemusterten Oberflache;
b) die gemusterte Oberfläche mit Fluorkohlenstoff plasma in Kontakt bringen, wobei ein hydrophobisches Material gezielt auf einen bestimmten Bereich der Oberfläche des Objekts eingelegt/abgelagert wird, während der Rest der Oberfläche des Objekts gleichzeitig geätzt wird;
c) ohne weitere Modifikationen außer der Aussetzung des Plasma wie in Schritt b) erklärt wird, die besagte in Plasma ausgesetzte gemusterte Oberfläche in Kontakt mit einer biomolekularen Lösung oder mit einer Vielfalt an biomolekularen Lösungen bringen, um die Adsorbierung der Biomoleküle zu ermöglichen, wobei das Biomolekül ein Protein ist.

2. Die Methode des Anspruchs 1, wobei das Substrat Si ist, und (wobei) der Rest der Oberfläche des Objekts silicium-dioxid SiO₂ oder silicium-nitrid Si₃N₄ ist.

3. Die Methode des Anspruchs1, wobei das Substrat Material Glas ist, und der gemusterte dünne Film lichtbeständig sind.

4. Irgendeine Methode der vorher erwähnten Ansprüche, wobei das Fluorkohlenstoff Plasma S₄F₈ oder eine Mischung aus CHF₃/CH₄ ist.

5. Irgendeine Methode der vorher erwähnten Ansprüche, wobei der Durchmesser der Messfleckgröße zwischen 100 nm und 1 mm liegt.

6. Die Methode des 5. Anspruchs, wobei der Durchmesser der Messfleckgröße zwischen 100 nm und 100 µm liegt.

7. Die Methode des Anspruchs 5, wobei der Durchmesser der Messfleckgröße zwischen 100 nm und 10 µm liegt.

8. Die Methode des Anspruchs 5, wobei der Durchmesser der Messfleckgröße zwischen 100 nm und 1 µm liegt.

9. Irgendeine Methode der vorher erwähnten Ansprüche, wobei Schritt b) mit Verwendung eines induktiv gekoppelten Plasma Reaktors durchgeführt wird.

10. Irgendeine Methode der vorher erwähnten Ansprüche, wobei in Schritt b) das Durchfluss- tempo der Fluorkohlenstoff Gase um die 25 sccm, der Gasdruck zwischen 2 und 10 mTorr, die Leistung zwischen 800 und 1500 Watt, die Verspannung zwischen -100 und -250 V, die Substrattemperatur zwischen -50° C und 0° C und die Bearbeitungszeit zwischen 10 und 90 Sekunden liegen.

11. Irgendeine Methode der vorher erwähnten Ansprüche, wobei in Schritt c die Ätzrate zwischen 70 und 270 nm/min für SiO₂ und zwischen 130 und 250 nm/min für Si₃N₄ liegt.

12. Irgendeine Methode der vorher erwähnten Ansprüche, wobei die Biomoleküle auf den hydrophilen Bereich adsorbiert werden.

13. Irgendeine Methode der vorher erwähnten Ansprüche, wobei die Aussetzung an ein Protein oder an einer peptiden Lösung von einem robotischen Spotter durchgeführt wird, wobei mehrere Proteine auf verschiedenen hydrophilen Bereichen adsorbiert oder getupft werden.

## Revendications

1. Une méthode pour la fabrication d'un micro-réseau de biomolécules sur un substrat présentant une surface pourvue d'un motif composé d'une pluralité de zones hydrophobes et hydrophiles, ladite méthode comprenant:
a) la fourniture d'un substrat présentant un motif sur sa surface;
b) le contact de ladite surface avec un plasma fluorocarboné au moyen duquel un matériau hydrophobique est sélectivement déposé sur des zones sélectionnées de ladite surface du substrat tandis que la partie restante de ladite surface du substrat est gravée;
c) sans aucune autre modification différente de celle de l'exposition au plasma de l'étape b) ci-dessus, le contact de ladite surface exposée au plasma avec une solution de biomolécules, ou avec une pluralité de solutions de biomolécules, afin de permettre l' adsoprtion de la biomolécule, où la biomolécule est une protéine.

2. La méthode de la revendication 1 dans laquelle le substrat est du silicium Si et ladite partie restante du substrat est du dioxyde de silicium SiO₂ ou du nitrure de silicium Si₃N₄.

3. La-méthode de la revendication 1 dans laquelle le matériau du substrat est du verre et le film fin doté d'un motif est une photorésine.

4. La méthode de n'importe quelle revendication précédente dans laquelle le plasma fluorocarboné est du C4F₈ ou une mixture de CHF₃/CH₄.

5. La méthode de n'importe quelle revendication précédente dans laquelle le spot a un diamètre allant d'environ 100nm à environ 1 mm.

6. La méthode de la revendication 5 dans laquelle le spot a un diamètre d'environ 100nm à environ 100 µm.

7. La méthode de la revendication 5 dans laquelle le spot a un diamètre allant d'environ 100nm à environ 10 µm.

8. La méthode de la revendication 5 dans laquelle le spot a un diamètre allant d'environ 100nm à environ 1 µm.

9. La méthode de n'importe quelle revendication précédente dans laquelle l'étape b) est réalisée en utilisant un réacteur à plasma à couplage inductif.

10. La méthode de n'importe quelle revendication précédente selon laquelle dans l'étape b), le débit du gaz fluorocarboné est d'environ 25 sccm, la pression du dit gaz est d'environ 2 à environ 10 mTorr, la puissance est d'environ 800 à environ 1500 Watt, la tension de polarisation est d'environ - 100 à environ -250 V, la température du substrat est d'environ -50 à environ 0 °C et la durée du procédé est d'environ 10 à environ 90 sec.

11. La méthode de n'importe quelle revendication précédente selon laquelle dans l'étape b), la vitesse de gravure est d'environ de 70 à 270nm/mn pour le SiO₂ et d'environ de 130 à 250 nm/mn pour le Si₃N₄.

12. La méthode de n'importe quelle revendication précédente dans laquelle la biomolécule est adsorbée sur la zone hydrophile.

13. La méthode de n'importe quelle revendication précédente dans laquelle l'exposition à une protéine ou une solution de peptides est effectuée au moyen de spots robotisés, dans lequel cas de multiples protéines peuvent être adsorbées ou déposées par spot sur différentes zones hydrophiles.
